# EUROPEAN PATENT APPLICATION

(11) **EP 4 732 826 A1**
(43) Date of publication of application: **29.04.2026**
(21) Application number: 24209095.9
(22) Date of filing: 28.10.2024
(51) Int. Cl.: A61K 8/365, A61K 8/368, A61K 8/44, A61Q 5/00, A61Q 5/12

(54) **HAIR CARE COMPOSITION COMPRISING SHIKIMIC ACID, ARGININE AND MALIC ACID**

(71) Applicant: SkyLab AG, 1066 Epalinges (CH)
(72) Inventor: Belous, Elena, 121309 MOSCOW (RU); Ivanova, Angelina, London, NW61NE (GB)
(74) Representative: Glawe, Delfs, Moll

(57) **Abstract**

The invention relates to a hair care composition comprising malic acid, arginine and shikimic acid, and to the use of said components in the manufacture of a hair care composition.

## Description

### FIELD OF THE INVENTION

The present invention pertains to novel applications of an organic monobasic monocyclic monounsaturated trihydroxy monocarboxylic acid - shikimic acid, an aliphatic basic α-amino acid - arginine, and an aliphatic 2-hydroxybutanedioic acid-malic acid. It is intended for use in the field of hair care formulations.

### BACKGROUND OF THE INVENTION

Hair is a derivative of the epidermis. Externally, it appears as thin, flexible shafts composed of dead, fully keratinized epithelial cells, while beneath the skin, it consists of individual live hair follicles - cylindrical epithelial projections extending into the dermis and subcutaneous fat, which expand at the base into the hair bulb, surrounding the mesenchymal dermal papilla [1].

Hair consists of two distinct structures: the follicle within the skin and the hair shaft, visible on the body's surface. The hair shaft is made up of the cortex and cuticular cells, and in some cases, a medulla located centrally. The medulla is the innermost region of the hair shaft, while the cortex, which constitutes the bulk of the hair fiber and plays a crucial role in its physico-mechanical properties, is a peripheral part consisting of approximately 50-60% macrofibrils. These macrofibrils are composed of microfibril rods embedded in a matrix [2]. The cuticle of the hair shaft covers the hair from root to tip and is formed by overlapping flat cells [3]. Each cuticle cell typically measures 0.3-0.5 µm in thickness and about 50 µm in visible length; it comprises various sub-lamellar structures (epicuticle, A-layer, exocuticle, endocuticle, inner layer) and a cell membrane complex [2]. The integrity and properties of the cuticular layer are vital in protecting the cortex from physical and chemical damage, maintaining the hair in a clean and manageable state, and significantly influencing its appearance [4].

The hair follicle is a crucial structure for hair growth. The follicle is surrounded by histological structures:
1) Outer Root Sheath (ORS), identified as a reservoir of multipotent stem cells, including keratinocyte and melanocyte stem cells, and containing keratinocytes. The ORS forms a distinct bulge region between the arrector pili muscle insertion point and the sebaceous gland duct [1,5]. On the dermal side, the ORS is bordered by a basket-like arrangement of two orthogonally oriented layers of collagen fibers - known as the glassy layer or dermal sheath .
2) The Inner Root Sheath (IRS) consists of three layers: Henle's layer, Huxley's layer, and the cuticular layer. The cuticular layer of the IRS adheres to the hair shaft cuticle, anchoring the hair shaft within the follicle. Cells of the IRS produce keratins and trichohyalin, which act as intracellular cement, providing strength to the IRS, supporting and shaping the growing hair shaft, and guiding its upward movement. The IRS separates the hair shaft from the ORS [1].

The primary function of the cuticle, with its rigid, inelastic protein content, is to protect the inner cortex, which ensures the elasticity of the hair. The hair cuticle is vital for shielding the internal structure, the cortex, from damage caused by environmental factors, cosmetic treatments, and industrial processes. It consists of several layers in human hair (a single layer in wool) of flattened, overlapping, and physically robust cells that cover the hair cortex. The outward orientation of the cell edges facilitates self-cleaning by continual epidermal exfoliation [8].

The cortex is composed of densely packed spindle-shaped cortical cells filled with keratin fibers oriented parallel to the longitudinal axis of the hair shaft and an amorphous matrix of high-sulfur proteins. The combination of an outer, intensely hydrophobic layer and the cortex imparts the physical properties of shine (luster) and body (volume), essential for the appearance of "healthy" hair [9].

Hair fiber degeneration is influenced by multiple mechanisms, such as friction, cosmetic products, wetting, heating, and ultraviolet radiation. In everyday life, all hair fibers experience cuticular and secondary cortical degradation to some extent before shedding. These changes, known as "weathering," are most characteristic of the tips of long hair, which are exposed to external stressors for extended periods. Progressive microscopic changes due to weathering include the lifting and uneven erosion of cuticle cells, leading to complete cuticle loss on some surface areas. Further damage results in longitudinal cracks between exposed cortical cells, followed by transverse splits (trichoschisis), trichorrhexis resembling nodules, and trichoptilosis (split ends) -a longitudinal splitting of the distal end of the hair shaft. In fact, the primary cause of trichorrhexis nodosa, also referred to as hereditary hair shaft fragility, is mechanical or chemical trauma. Bleached hair has an abnormal texture, volume, and shine, making it difficult to manage [10].

Exposure to sunlight leads to dryness, surface roughness, reduced shine, stiffness, and brittleness of the hair, as well as changes in its color. Transmission electron microscopy of hair exposed to sunlight reveals the breakage and detachment of the outer cuticle layers or even complete destruction of the cuticle layer and split ends. The cuticle is more prone to damage than the cortex because it lacks photoprotective melanin granules and is exposed to higher intensities of radiation. Damage to the proteins and lipids within the hair cuticle is primarily caused by ultraviolet radiation (UVA and UVB) and, to a lesser extent, by visible light. The adverse effects of sun exposure are amplified by moisture and humidity, which is why "surfer's hair," subjected to sun and saltwater, is known to be brittle, damaged, and discolored. The loss of hair color results from damage to melanin granules, mainly due to exposure to visible light [11].

Silicones have been used in personal care products since the 1950s. Initially included in skincare products and more recently in hair care products, silicones gained recognition for their lubricating properties and the distinct soft, smooth feel they impart. Thanks to recent advancements in silicone technology, these fluids can also provide durability and longevity. Resinous silicones, such as trimethylsiloxysilicate, act as effective auxiliary additives when combined with dimethicone in skincare formulations. In tests evaluating the number of wash cycles required to penetrate through the silicone barrier, resistance to removal noticeably increases as the proportion of resinous silicone relative to dimethicone rises. This improvement is directly linked to the reduced solubility of the resin. Concerns about the potential risks of silicone stem from the fact that high-polymer silicone oils or resins create a non-washable film on the hair surface. On one hand, this film provides excellent protection against aggressive factors, but on the other, such silicones are not removed by water, and if the hair is not thoroughly cleaned for an extended period, it becomes brittle, fragile, and lifeless. This is especially noticeable on fine hair - the hairstyle quickly loses volume, and the strands appear greasy [12].

Regarding the product Olaplex, its active ingredient is bis-aminopropyl diglycol dimaleate. Experimental studies have proven that this component protects hair during penning, chemical coloring, straightening, and other harsh treatments by building protection in the form of so-called "disulfide bridges." Thanks to this protection, the hair strands not only retain their original qualities but even acquire new ones: smoothness, elasticity, resilience, silkiness, and a healthy shine. OLAPLEX No. 1 is a liquid containing the active ingredient. The solution is mixed with the dye or applied to the hair before coloring. The product rebuilds the "bridges" in damaged disulfide bonds, thereby protecting the hair structure from harmful chemical dyes. OLAPLEX No. 2 is a sort of fixing cocktail. It reinforces the action of the first solution and is used until the hair is fully restored. The second solution is dominated by components essential for moisturizing and softening the hair: vitamins, proteins, plant extracts, and natural oils. OLAPLEX No. 3 is designed for at-home care. It contains therapeutic components and is applied once a week. There are three cases in which the effectiveness of this product is reduced:
1) If hair falls out and splits due to aging, it is best to select a specialized anti-aging product.
2) If the strands have been damaged by chemical perming or have lost their shine due to constant lightening.
3) The use of gentle hair dyes (free of ammonia, MEA, ethanolamine) does not disrupt disulfide bonds. Therefore, the need for OLAPLEX in these cases is reduced.
The products in the Olaplex system contain an active ingredient that works at the molecular level. It reconnects broken disulfide bonds within the hair structure that have been damaged during adverse chemical, thermal, or mechanical treatments [13].

Numerous studies have discussed the properties of essential oils and the fact that each essential oil possesses a set of potential therapeutic effects based on its chemical composition. These therapeutic properties are determined by the chemical makeup, where typically the main components reflect not only the biophysical but also the biological characteristics of the essential oil from which they are derived. However, the synergistic effect of several molecules within essential oils remains unclear compared to the effects of just one or two primary isolated components.

The field of cosmetic dermatology is growing due to the connection between medical hair treatments and traditional cosmetology. As a result, essential oils are becoming increasingly popular in cosmetic and therapeutic hair products because of the growing number of reports on their beneficial effects on the scalp and hair. It has been reported that a few drops of essential oils can be used during the final rinse or added directly to shampoo. Essential oils such as rosemary (Rosmarinus officinalis) and chamomile (Matricaria chamomilla) help condition and stimulate hair growth; lavender (Lavandula angustifolia) can be used to repel lice and fleas, while bergamot (Citrus bergamia) and tea tree (Melaleuca alternifolia) can combat dandruff. Some essential oils can penetrate the hair follicle and bind to receptors that stimulate hair growth, making them one of the safest options for treating alopecia. However, therapeutic interventions using essential oils are still not well-documented in the scientific literature [14].

Hair exhibits dual properties of proteins and amino acids, and both can be used in hair care formulations [15]. Amino acids such as serine have a certain affinity for hair and act as nutritionally active substances as well as moisturizing ingredients that can help restore damaged hair. Some commercial hair conditioners contain added amino acids, but their concentration is low, and they can be easily washed away. Both human hair and wool are primarily composed of α-keratin, and the amino acid composition of the fibers is similar in each case [16]. Therefore, it can be expected that results observed in wool restoration may also apply to human hair [17]. The study [18] found that amino acids with homology bind to amino acid residues on the molecular chain of wool fibers, and simultaneously, the macromolecular chains of broken wool fibers are reconnected and cross-linked, improving the characteristics of fragile wool fabrics. In 2021, a study [19] utilized low-concentration keratin with the enzyme transglutaminase for targeted catalysis of more covalent cross-linking of keratin to repair damaged hair samples. The authors aimed to investigate the effect of cross-links between amino acids and proteins with the help of transglutaminase on hair care. Compared to traditional hair care products, which typically add only keratin or amino acids, the keratin-serine-enzyme repair solution described here [20] offers the potential for fundamental improvement and restoration of hair structure. In this method, keratin and serine form a protective film through the catalytic action of transglutaminase, which can repair major damaged parts of the hair using internal keratin and supplement α-keratin in the damaged cortex. Serine leverages its small molecular mass to unite and repair damaged sections in thinner areas, thereby altering the physical and chemical properties of the hair, increasing the amide bond content in damaged hair, improving crystallinity, enhancing the thermal stability of damaged hair, and restoring the cuticles on the surface, giving damaged hair a smoother appearance. This reduces the water loss rate in damaged hair, making it more elastic, shiny, healthy, and attractive. Products of this type not only effectively restore damaged hair but also prevent damage to samples of healthy hair.

Oxidizing agents also mechanically weaken hair by oxidizing cysteine residues into cysteic acid, which breaks the disulfide bridges that typically form between two cysteine residues. The two most significant changes in the amino acid composition of bleached versus non-bleached hair are the reduction in half of the cystine residues and an increase in cysteic acid residues [21].

Arginine, as an amino acid, can be utilized in various formulations as an active ingredient. Normal hair contains about 10.5% arginine by mass; arginine-deficient hair tends to be weak and prone to breakage [22]. Specifically, the benefits of arginine for hair are attributed to its ability to: enhance microcirculation in the blood vessels and improve the blood supply to hair follicles; accelerate the delivery of nutrients to hair roots; boost the barrier function of the epidermis; facilitate the removal of toxins from skin cells; cleanse the epidermis of protein breakdown products, normalizing its acid-base balance; speed up hair growth and restore its structure (the effects become visible as hair grows out); stimulate the regeneration of damaged tissues and promote cell rejuvenation. It is reported that arginine plays crucial roles: it protects the skin from free radicals, acts as an antioxidant, stimulates collagen production, is involved in cell division, and aids in the healing process. Arginine can also act as a humectant, increasing the hydration of the stratum corneum [23].

Malic acid is primarily produced for use in the food industry as an acidulant and flavor enhancer, labeled as E296 [24]. However, its use in cosmetics, including hair care products, as a fragrance and pH regulator has become more common [25]. Malic acid has been reported to improve the strength of hair under humid conditions. This is attributed to its ability to form numerous hydrogen bonds with the proteins of hair fibers, thereby reducing the moisture content in the hair [26]. The study in [27] confirmed that malic acid effectively suppresses light scattering from the medulla, giving dull hair a clean and shiny appearance, and reduces the formation of shiny specks associated with thermal hair treatments, which result from the splitting of cuticle layers. However, the use of malic acid in products is limited by its side effects. Although milder than other alpha hydroxy acids, malic acid may still cause skin irritation. When used as an additional component in formulations, it has been associated with redness, peeling, dryness, and inflammation. These factors highlight potential issues when using malic acid in combination with other substances [Soumya Tamatam. Why You Need To Add Malic Acid To Your Skin Care Right Away. Available online: https://skinkraft.com/blogs/articles/malic-acid#:~:text=Side%20Effects%200f%20Ma1ic%20Acid,-Even%20though%20malic&text=Besides%2C%20malic%20acid%20is%20used,test%20iso/Q 20recommended%20before%20usage].

Shikimic acid, or shikimate, is primarily used in the pharmaceutical industry for drug production; however, it has recently gained interest in the cosmetics industry. Its most significant properties include the restoration of delicate tissues by repairing the strongest disulfide -S-S- bonds in keratin and silk structures, enhancing the strength and smoothness of fibers, sealing cuticles (which helps retain dye within fiber structures), creating a hydrophobic effect, eliminating unpleasant odors, providing antibacterial effects, regulating microflora, and promoting hair growth. It has also been proposed as a new therapeutic agent for treating alopecia [28]. Given the versatile nature of shikimic acid and its expanding applications across various fields, it is evident that this biobased material warrants further research and use as a renewable and reliable resource for the modern chemical and pharmaceutical industries [29].

Thus, the hair structure restoration technology acts on all levels of hair damage. Shikimic acid repairs disulfide bonds, arginine works on the hair surface (the cuticle), and malic acid forms hydrogen bonds, giving hair a clean and shiny appearance [26, 27, 30].

### Experiment 1. In vitro determination of the efficacy of the complex for hair cuticle restoration.

### 1.1 Materials and methods

### Hair Sample Preparation

Natural hair tresses were selected for this study, characterized by a cool white blonde coloration to ensure uniformity across all samples. Each tress was pre-treated to align with the baseline color and texture properties, thus ensuring consistency in the assessment of treatment effects. Commercial protease was bought from Creative Enzymes, USA, as an ingredient for targeted destruction of hair cuticle. Investigated ingredients for hair restoration presented in the Table 1.

**Table 1. Investigated ingredients**

| No. | INCI | CAS Number | Origin |
|---|---|---|---|
| 1 | Malic acid | 97-67-6 | Natural origin |
| 2 | Arginine | 74-79-3/7200-25-1 | Synthetic |
| 3 | Shikimic acid | 138-59-0 | Natural origin |
| 4 | Keratin | 68238-35-7 | Natural origin |

### 1.2 Investigation

Cuticular Surface Analysis: The cuticular morphology of the first hair tress was subjected to Scanning Electron Microscopy (SEM) to establish a baseline structural profile.

Proteolytic Solution Formulation: An aqueous solution with a 0.25% concentration of a commercially sourced protease was prepared, involving the dissolution of 0.75 grams of the enzyme in 300 milliliters of distilled water, followed by homogenization to ensure uniform distribution.

Proteolytic Treatment Incubation: The remaining thirteen tresses (excluding the first) were submerged in the proteolytic bath. The solution was agitated at 15-minute intervals using a Pasteur pipette over a period of one hour to facilitate enzymatic penetration and action.

Post-Treatment Rinse and SEM Assessment: Post enzymatic exposure, tresses numbered 2 through 14 were lavaged with copious amounts of water. Subsequent to this, the second hair tress underwent SEM to evaluate the enzymatic impact on cuticular integrity.

Subsequent Treatment Protocols: Twelve tresses from the previous step were allocated for further treatment with varying concentrations of active ingredients:
- As a positive control a solution with 1.5% keratin was prepared in distilled water. This preparation was adjusted to a pH range of 5-6, corresponding to hair care product standards. Keratin was used as a well-known ingredient for hair restoration which is biomimetic to hair cuticle. Hair tress number 3 was immersed in this bespoke solution for a duration of one hour.
- An isotonic solution was prepared, containing 0.1% shikimic acid, 0.1% arginine, and 0.1% malic acid in distilled water. This preparation was adjusted to a pH range of 5-6, corresponding to hair care product standards. Hair tress number 4 was immersed in this bespoke solution for a duration of one hour.
- An isotonic solution was prepared, containing 0.2% shikimic acid, 0.2% arginine, and 0.2% malic acid in distilled water. This preparation was adjusted to a pH range of 5-6, corresponding to hair care product standards. Hair tress number 5 was immersed in this bespoke solution for a duration of one hour.
- An isotonic solution was prepared, containing 0.5% shikimic acid, 0.5% arginine, and 0.5% malic acid in distilled water. This preparation was adjusted to a pH range of 5-6, corresponding to hair care product standards. Hair tress number 6 was immersed in this bespoke solution for a duration of one hour.
- A solution was concocted with modified concentrations of 0.5% shikimic acid, 0.5% arginine, and 0.2% malic acid in distilled water. Upon solute integration, pH adjustments were made to achieve a range of 5-6. Tress number 7 was then incubated in this solution for one hour.
- Lastly, a hyperconcentrated mixture containing 1% shikimic acid, 1% arginine, and 0.5% malic acid was developed. The solution, after addition of the respective solutes to distilled water, was pH-balanced to hair care product standards, followed by a one-hour immersion of hair tress number 8.
- A solution of 0.1% shikimic acid was prepared to visualize the effects of a specific component of the complex and to deduce the potential presence of synergistic interactions. Hair tress number 9 was immersed in this bespoke solution for a duration of one hour.
- A solution of 0.1% arginine was prepared to visualize the effects of a specific component of the complex and to deduce the potential presence of synergistic interactions. Hair tress number 10 was immersed in this bespoke solution for a duration of one hour.
- A solution of 0.1% malic acid acid was prepared to visualize the effects of a specific component of the complex and to deduce the potential presence of synergistic interactions. Hair tress number 11 was immersed in this bespoke solution for a duration of one hour.
- A 0.1% Arginine + 0.1% Shikimic acid solution was prepared to visualise the effects of two of the three components of the complex and to infer whether the third component should be added. Hair tress number 12 was immersed in this bespoke solution for a duration of one hour.
- A 0.1% Arginine + 0.1% Malic acid solution was prepared to visualise the effects of two of the three components of the complex and to infer whether the third component should be added. Hair tress number 13 was immersed in this bespoke solution for a duration of one hour.
- A 0.1% Shikimic acid + 0.1% Malic acid solution was prepared to visualise the effects of two of the three components of the complex and to infer whether the third component should be added. Hair tress number 14 was immersed in this bespoke solution for a duration of one hour.

The pH of all solutions was adjusted to 5.5, which is physiologically appropriate for the hair cuticle.

After a thorough rinsing of tresses numbered 3 to 14, all following samples from Table 2 were employed for a meticulous examination of the cuticular alterations post-chemical treatment by Scanning Electron Microscopy (SEM). For SEM analysis, samples were extracted from the median section of each hair tress to ensure representativeness. The microstructural examination of the acquired materials was conducted using a Tescan Amber GMH scanning electron microscope at an accelerating voltage of 1 kV, with magnifications ranging from 250x to 1000x to capture detailed topographical information of the hair cuticle.

**Table 2. Investigated systems**

| NO. | TREATMENT GROUP | SEM IMAGES OBTAINED (QUANTITY) |
|---|---|---|
| 1 | Positive control - untreated hair | 21 |
| 2 | Negative control - treated with protease | 21 |
| 3 | 1.5% Keratin | 18 |
| 4 | 0.1% Shikimic acid, 0.1% Arginine, 0.1% Malic acid | 16 |
| 5 | 0.2% Shikimic acid, 0.2% Arginine, 0.2% Malic acid | 17 |
| 6 | 0.5% Shikimic acid, 0.5% Arginine, 0.5% Malic acid | 17 |
| 7 | 0.5% Shikimic acid, 0.5% Arginine, 0.2% Malic acid | 18 |
| 8 | 1% Shikimic acid, 1% Arginine, 0.5% Malic acid | 25 |
| 9 | 0.1% Shikimic acid | 17 |
| 10 | 0.1% Arginine | 21 |
| 11 | 0.1% Malic acid | 19 |
| 12 | 0.1% Arginine + 0.1% Shikimic acid | 16 |
| 13 | 0.1% Arginine + 0.1% Malic acid | 20 |
| 14 | 0.1% Malic acid + 0.1% Shikimic acid | 20 |

### 1.3 Results and discussion

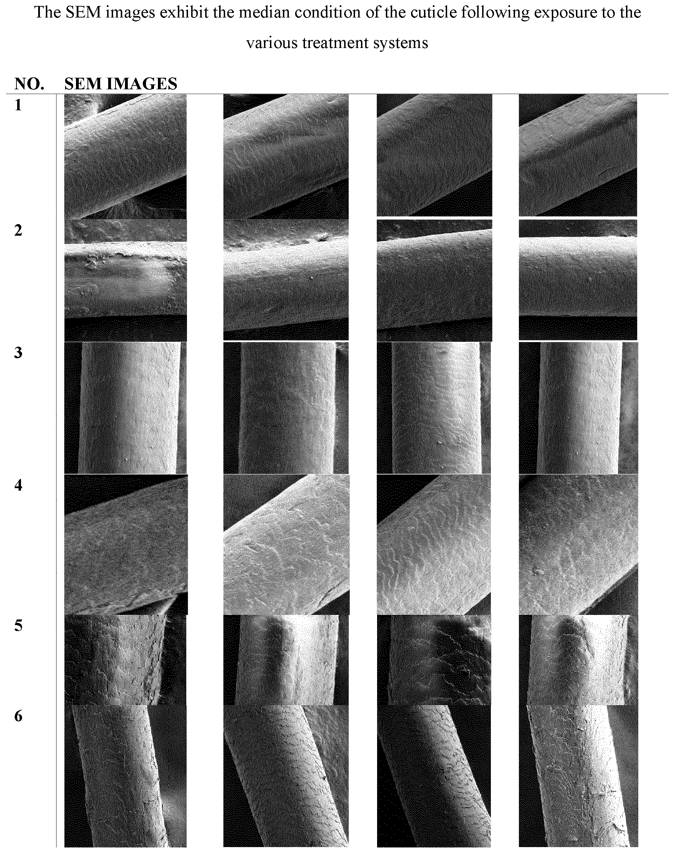

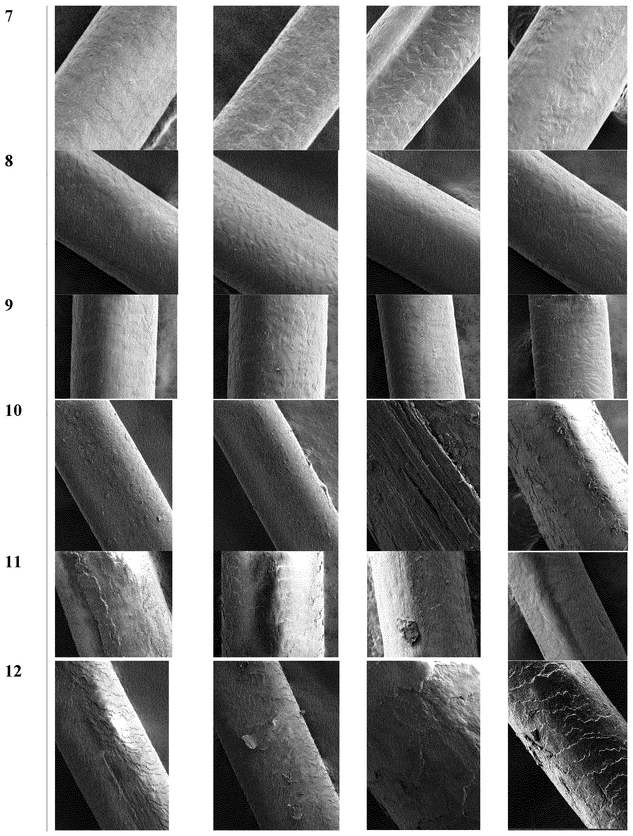

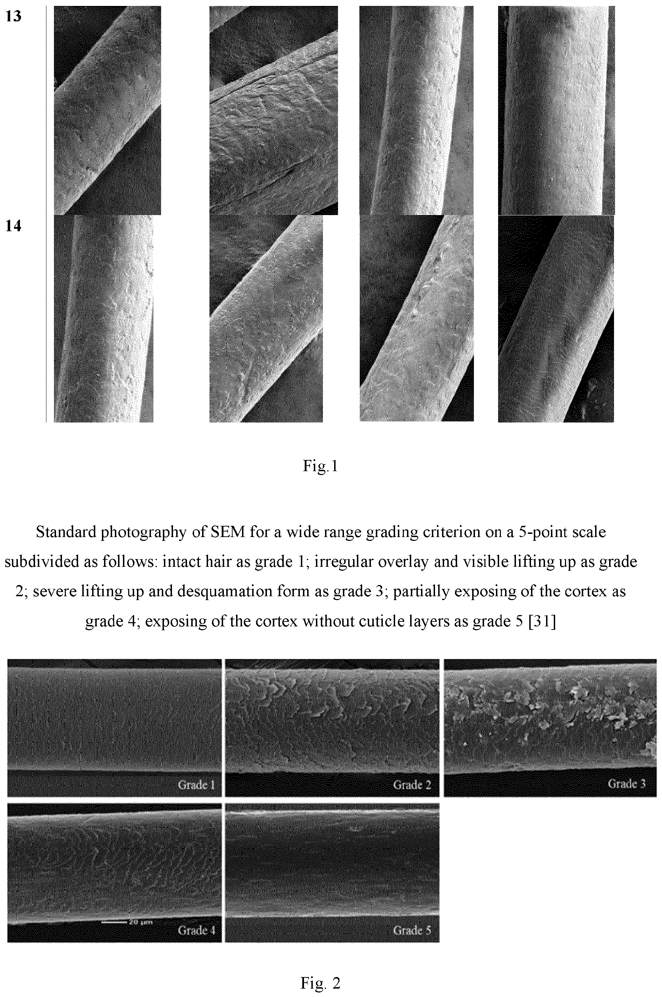

Following the Scanning Electron Microscopy (SEM) examination, a series of 16 to 25 images was captured for each sample, documenting the condition of the cuticle across a selection of hairs within the analyzed test tress. Condition of hair cuticles were analyzed using method illustrated in Figure 2. To facilitate the evaluation, the outcome data for each treatment cohort was quantified using the 'Hair Cuticle Restoration Index,' where a rating of 1 denotes the optimal state of cuticle integrity and a rating of 5 represents the most compromised condition.

**Table 3. Hair cuticle restoration index for treatment groups**

| NO. | SYSTEM | HAIR CUTICLE RESTORATION INDEX | STDEV |
|---|---|---|---|
| 1 | Positive control - untreated hair | 1.42 (p < 0.05) | 1.12 |
| 2 | Negative control - treated with 0.25% protease | 4.32 (p < 0.05) | 0.51 |
| 3 | 1.5% Keratin | 2.33 (p < 0.05) | 0.64 |
| 4 | 0.1% Shikimic acid, 0.1% Arginine, 0.1% Malic acid | 1.87 (p < 0.05) | 0.65 |
| 5 | 0.2% Shikimic acid, 0.2% Arginine, 0.2% Malic acid | 1.98 (p < 0.05) | 0.61 |
| 6 | 0.5% Shikimic acid, 0.5% Arginine, 0.5% Malic acid | 2.27 (p < 0.05) | 0.58 |
| 7 | 0.5% Shikimic acid, 0.5% Arginine, 0.2% Malic acid | 3.75 (p < 0.05) | 0.71 |
| 8 | 1% Shikimic acid, 1% Arginine, 0.5% Malic acid | 3.66 (p < 0.05) | 0.79 |
| 9 | 0.1% Shikimic acid | 3.08 (p < 0.05) | 0.72 |
| 10 | 0.1% Arginine | 3.98 (p < 0.05) | 0.43 |
| 11 | 0.1% Malic acid | 4.14 (p < 0.05) | 0.50 |
| 12 | 0.1% Arginine + 0.1% Shikimic acid | 3.22 (p < 0.05) | 0.74 |
| 13 | 0.1% Arginine + 0.1% Malic acid | 4.01 (p < 0.05) | 0.66 |
| 14 | 0.1% Malic acid + 0.1% Shikimic acid | 3.51 (p < 0.05) | 0.72 |

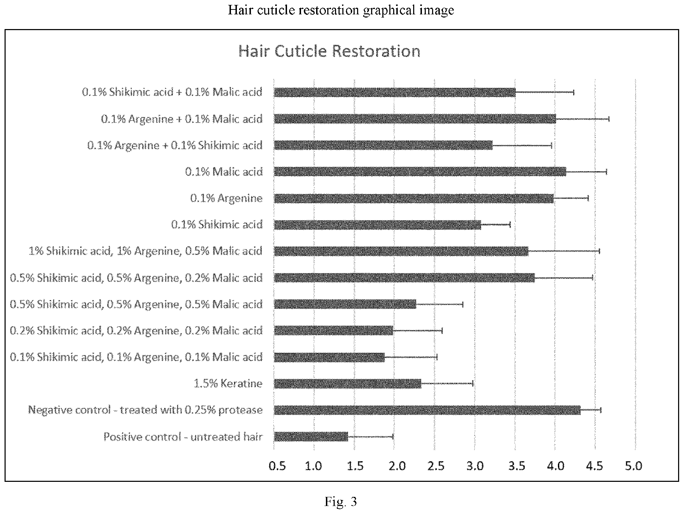

The results detailed in Table 3 and illustrated in Figure 3 demonstrate the outcomes of the study. The undamaged test strand No. 1, serving as the negative control, exhibited the healthiest cuticle condition with a mean Hair Cuticle Restoration Index value of 1.42. The degradation of test strand No. 2 by protease significantly compromised the cuticle integrity, reflected in a quality index value of 4.32. Keratin, employed as a market reference biomimetic to hair for restorative purposes, yielded a cuticle recovery result of 2.33, equating to a 64% restoration. A compound mix of shikimic acid, arginine, and malic acid, introduced as a restorative agent at three different concentrations, revealed varying efficacies. At the highest concentrations used-1% shikimic acid, 1% arginine, and 0.5% malic acid-the cuticle of hair strand No. 8 experienced a 22% restoration. Intermediate concentrations of 0.5% shikimic acid, 0.5% arginine, and 0.2% malic acid resulted in a 20% restoration of the cuticle for hair strand No. 7, indicating marginally lesser effectiveness compared to the higher concentration compound. However, the most significant efficacy was observed at the lowest concentrations of 0.1% for shikimic acid, arginine, and malic acid, which led to an 84% cuticle restoration of hair strand No. 4 compared to the initial condition, surpassing the keratin market benchmark by 20%. Good restoration results were demonstrated by solution of restoration for 0.2% shikimic acid, 0.2% arginine, and 0.2% malic acid, which led to an 81% cuticle restoration of hair strand No. 5 and by solution of restoration for 0.5% shikimic acid, 0.5% arginine, and 0.5% malic acid, which led to a 71% cuticle restoration of hair strand No. 6. This finding could be primarily attributed to the effectiveness of the compound at lower concentrations. Given that the restorative treatments for strands No. 7 and No. 8 component ratio of 1:1:0.5, the substantial contribution of malic acid to the hair structure's restoration can be inferred. Shikimic acid, utilized as a singular ingredient, exhibited a cuticle repair efficacy score of 3.08, indicating a recovery rate of 43%. Arginine, utilized as a singular ingredient, exhibited a cuticle repair efficacy score of 3.98, indicating a recovery rate of 11,6%. Malic acid, utilized as a singular ingredient, exhibited a cuticle repair efficacy score of 4.14, indicating a recovery rate of 6,1%. This outcome is inferior to that achieved by the composite formulation of samples No. 4-6, thereby providing further evidence of the synergistic interactions among its constituents. Reconstitution with combinations of two components which were carried out to visualize the effects of two of the three components of the complex and to infer whether the third component should be added showed the following results: reconstitution of 0.1% shikimic acid and 0.1% arginine showed a 38% recovery; reconstitution of 0.1% shikimic acid and 0.1% malic acid showed a 28% recovery; 0.1% arginine and 0.1% malic acid showed a 11% recovery. These results suggest that a third component should be added to obtain a synergistic effect.

An important aspect of the study's findings is the cost-effectiveness related to the formulation concentrations; a composite of the 0.1% of each active ingredients surpassed the performance of 1.5% keratin, potentially influencing the final product's price point. Additionally, the system under investigation is vegan, in contrast to keratin, which lacks Vegan or Vegetarian certification.

### Experiment 2. Hair disulfide bonds restoration

In this experimental study, standardized bundles of light-colored hair were utilized to investigate the effect of hydrogen peroxide on disulfide bond integrity within the hair's structural matrix. Each hair bundle underwent a treatment regimen with a solution of peroxide, known for its oxidative capabilities to disrupt disulfide linkages [32]. The hair samples were subjected to a one-hour exposure to the thioglycolic acid solution to ensure adequate interaction time for bond disruption.

Following the thioglycolic acid treatment, the hair was thoroughly rinsed with distilled water to remove residual oxidizing agent. One sample from the treated batch was set aside to serve as a negative control, characterized by the disrupted disulfide bonds. In contrast, a subsequent sample was designated for treatment with a restorative system.

The restorative system was prepared by dissolving 1 gram of shikimic acid (INCI: Shikimic Acid; CAS: 138-59-0), 1 gram of arginine (INCI: Arginine; CAS: 74-79-3/7200-25-1), and 0.5 grams of malic acid (INCI: Malic Acid; CAS: 97-67-6) into 100 milliliters of distilled water, thus achieving a 1%, 1%, and 0.5% solution, respectively. The preparation of the solution was conducted under ambient conditions, with the constituents being added sequentially while stirring continuously to ensure homogeneity of the solution. The pH was monitored and adjusted to the physiological range optimal for hair cuticle interaction.

The second hair sample, now with disrupted disulfide bonds from the prior peroxide treatment, was then immersed in the freshly prepared restorative solution for a period of one hour. This process was designed to evaluate the potential reconstitution effects of the active agents on the hair's damaged disulfide bonds.

**Table 4. Investigated hair tresses.**

| No. | Samples description |
|---|---|
| 1 | Hair tressed with destroyed disulfide bonds with peroxide 2% |
| 2 | Hair tresses with recovered disulfide bonds with system |

After treatments analytical evaluation of disulfide bonds were executed utilizing a Confotec NR500 laser confocal Raman microscope (Solar Instruments Ltd, Belarus). The methodological approach involved continuous wave laser irradiation at a wavelength of 785 nm with an emission power set at 24 mW. This beam was meticulously focused onto the samples through an Olympus optical lens system featuring a numerical aperture of 0.45 and a magnification factor of 20x. The spectral data accumulation was systematically performed for a duration of 5 seconds across 5 iterations. This process yielded a comprehensive spectral distribution in the wavenumber region spanning from 300 to 1800 cm^-1.

**Table 5. Interpretation and description of oscillations in the obtained Raman spectrum.**

| *Position, cm(-1)* | *Decoding* |
|---|---|
| *501* | S - S |
| *1 003* | Phenylalanine |
| *1 126* | C-N |
| *1 176* | Tyrosine |
| *1 248* | Amide (III) |
| *1 342* | CH2 |
| *1 663* | Amide (I) |

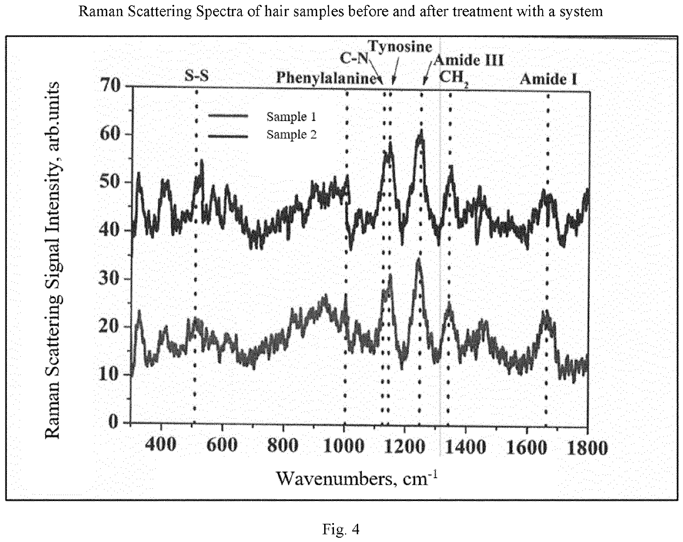

The resultant Raman scattering spectrum for Sample 1 comprised multiple vibrational modes as delineated in Table 5, consistent with established spectral characteristics commonly attributed to hair fiber analysis, encompassing both carbonaceous moieties (C-N, CH2) and amino acid residues (phenylalanine, tyrosine, and various amide linkages) [33]. Consequential to the cosmetic intervention, Sample 2 exhibited distinctive spectral signatures indicative of newly formed disulfide cross-links (Figure 4), conspicuously marked by an emergent peak proximal to the wavenumber of 509 cm^-1, which serves as a spectral hallmark for the presence of S-S bond formation within the hair's structure.

### LITERATURE

1. Randall VA, Botchkareva NV. The biology of hair growth. In: Ahluwalia GS, ed. Cosmetic Application of Laser and Light-Based System. Norwich, NY: William Andrew Inc., 2009: 3-35.
2. Wolfram LJ. Human hair: a unique physicochemical composite. J Am Acad Dermatol 2003; 48: S106- S114.
3. Gurden SP, Monteiro VF, Longo E, et al. Quantitative analysis and classification of AFM images of human hair. J Microsc 2004; 215: 13-23.
4. Swift JA. Human hair cuticle: biologically conspired to the owners advantage. J Cosmet Sci 1999; 50: 23-47.
5. Oshima H, Rochat A, Kedzia C, et al. Morphogenesis and renewal of hair follicles from adult multipotent stem cells. Cell 2001; 104: 233-245.
6. Rogers GE. Electron microscope observations on the glassy layer of the hair follicle. Exp Cell Res 1957; 13: 521-528.
7. Rogers GE. Hair follicle differentiation and regulation. Int J Dev Biol 2004; 48: 163-170.
8. Rogers, G.E. Known and Unknown Features of Hair Cuticle Structure: A Brief Review. Cosmetics 2019, 6, 32.
9. Rodney D. Sinclair. Healthy Hair: What Is it? \\ Journal of Investigative Dermatology Symposium Proceedings, Volume 12, Issue 2, 2007, Pages 2-5.
10. Horev, L. (2007). Environmental and Cosmetic Factors in Hair Loss and Destruction. Current Problems in Dermatology, 103-117.
11. Takahashi T, Nakamura K: A study of the photolightening mechanism of blond hair with visible and ultraviolet light. J Cosmet Sci 2004; 55:291-305.
12. DISAPIO, A., & FRIDD, P. (1988). Silicones: use of substantive properties on skin and hair. International Journal of Cosmetic Science, 10(2), 75-89.
13. http://www.olaplex.ru/how-it-works/
14. Abelan, U. S., Oliveira, A. C., Cacoci, É. S. P., Martins, T. E. A., Giacon, V. M., Velasco, M. V. R., & Lima, C. R. R. de C. (2021). Potential use of essential oils in cosmetic and dermatological hair products: A review. Journal of Cosmetic Dermatology.
15. Cruz, C.F.; Azoia, N.G.; Matamá, T.; Cavaco-Paulo, A. Peptide-Protein interactions within human hair keratins. Int. J. Biol. Macromol. 2017, 101, 805-814.
16. Soun, B.; Kaur, D.; Jose, S. Effect of Transglutaminase Enzyme on Physico-mechanical Properties of Rambouillet Wool Fiber. J. Nat. Fibers 2020, 17, 793-801.
17. Lin, Z. Study on mTG Enzyme-Catalyzed Reinforcement of Fragile Wool Fabrics. Ph.D. Thesis, Zhejiang Sci-Tech University, Hangzhou, China, 2016.
18. Li, Z.; Xiao, J. Repair of damaged hair protein fiber by jointly using transglutaminase and keratin. Scienceasia 2021, 47, 195.
19. Gillespie, J.M.; Haylett, T.; Lindley, H. Evidence of homology in a high-sulphur protein fraction (SCMK-B2) of wool and hair alpha-keratins. Biochem. J. 1968, 110, 193-200.
20. Liu, Y.; Liu, J.; Xiao, J. Enzymatic Crosslinking of Amino Acids Improves the Repair Effect of Keratin on Hair Fibre. Polymers 2023, 15, 2210.
21. Luke I. Chambers, Dmitry S. Yufit, Osama M. Musa, and Jonathan W. Steed \\ Crystal Growth & Design 2022 22 (10), 6190-6200.
22. Sandesh C.S. Nagamani, Ayelet Erez, Brendan Lee. Argininosuccinate lyase deficiency \\ Genetics in Medicine, Volume 14, Issue 5, 2012, Pages 501-507.
23. Almeida, C.; Rijo, P.; Rosado, C. Bioactive Compounds from Hermetia Illucens Larvae as Natural Ingredients for Cosmetic Application. Biomolecules 2020, 10, 976.
24. Kövilein A. et al. Malic acid production from renewables: a review //Journal of chemical technology & biotechnology. - 2020. - T. 95. - No. 3. - C. 513-526.
25. Burnett C. L. et al. Amended Safety Assessment of Malic Acid and Sodium Malate as Used in Cosmetics //International journal of toxicology. - 2022. - T. 41. - No. 3_suppl. - C. 69-76.
26. Itou T, Nojiri M, Ootsuka Y, Nakamura K. Study of the interaction between hair protein and organic acid that improves hair-set durability by near-infrared spectroscopy. J Cosmet Sci. 2006; 57(2): 139-151.
27. Okamoto, M.; Yakawa, R.; Mamada, A.; Inoue, S.; Nagase, S.; Shibuichi, S.; Kariya, E.; Satoh, N. Influence of internal structure of hair fiber on hair appearance. III. Generation of light-scattering factors in hair cuticles and the influence on hair shine. J. Cosmet. Sci. 2003, 54, 353-366.
28. Choi, M., Choi, S.-J., Jang, S., Choi, H.-I., Kang, B.-M., Hwang, S. T., & Kwon, O. (2019). Shikimic acid, a mannose bioisostere, promotes hair growth with the induction of anagen hair cycle. Scientific Reports, 9(1).
29. Rawat, G., Tripathi, P. & Saxena, R.K. Expanding horizons of shikimic acid. Appl Microbiol Biotechnol 97, 4277-4287 (2013).
30. Michele Di Foggia, Carla Boga, Gabriele Micheletti, Benedetta Nocentini, Paola Taddei. Structural investigation on damaged hair keratin treated with α,β-unsaturated Michael acceptors used as repairing agents \\ International Journal of Biological Macromolecules, Volume 167, 2021, Pages 620-632.
31. Lee S. Y. et al. Twelve-point scale grading system of scanning electron microscopic examination to investigate subtle changes in damaged hair surface //Skin Research and Technology. - 2016. - T. 22. - No.4. - C. 406-411.
32. Wickett RR. Permanent waving and straightening of hair. Cutis. 1987 Jun;39(6):496-7. PMID: 3608576.
33. Jordana Dias dos Santos,Howell G.M. Edwards,Luiz Fernando Cappa de Oliveira. Raman spectroscopy and electronic microscopy structural studies of Caucasian and Afro human hair. Elsevier. 2019 May; 5(5)

## Claims

1. A hair care composition comprising the following components A, B and C:
A) malic acid,
B) arginine, and
C) shikimic acid;
wherein in said hair care composition each of said components A, B and C is comprised by <_ 0.5% by weight of said composition.

2. The hair care composition of claim 1, wherein in said hair care composition each of said components A, B and C is comprised by at least 0.01%, preferably at least 0.05%, further preferably at least 0.1% by weight of the composition.

3. The hair care composition of claim 1 or claim 2, wherein the mass ratio of said components A, B and C in said hair care composition is selected from one of the following options a)-f):
option a)
| A | : | B | : | C |
|---|---|---|---|---|
| 0.01-0.5 | | 0.01-0.5 | | 0.01-0.5 |
option b)
| A | : | B | : | C |
|---|---|---|---|---|
| 0.05-0.5 | | 0.05-0.5 | | 0.05-0.5 |
option c)
| A | : | B | : | C |
|---|---|---|---|---|
| 0.1-0.5 | | 0.1-0.5 | | 0.1-0.5 |
option d)
| A | : | B | : | C |
|---|---|---|---|---|
| 0.01-0.2 | | 0.01-0.2 | | 0.01-0.2 |
option e)
| A | : | B | : | C |
|---|---|---|---|---|
| 0.05-0.2 | | 0.05-0.2 | | 0.05-0.2 |
option f)
| | | | | |
|---|---|---|---|---|
| A | : | B | : | C |
| 0.1-0.2 | | 0.1-0.2 | | 0.1-0.2 |

4. The hair care composition of any of the preceding claims, wherein said components A, B and C are comprised in said composition in the identical % by weight, wherein preferably each of said components A, B and C is comprised in said composition by 0.5, 0.2 or 0.1 % by weight of said composition.

5. The hair care composition of any of the preceding claims, wherein said hair care composition is assembled as single composition comprising each of said components A, B and C, or as a kit-of-parts composition separating at least one of said components A, B and C from the remining components, and wherein said parts of said kit-of-parts are for combining said components prior use.

6. The hair care composition of any of the preceding claims, wherein said hair care composition is formulated for application on a subject's hair.

7. The hair care composition of any of the preceding claims, wherein said hair care composition is a formulation selected from the following: liquid, aerosol, suspension, solution, tincture, cream, paste, lotion, ointment, gel, balsam, foam, wherein said composition is preferably a shower gel and/or shampoo.

8. The hair care composition of any of the preceding claims, wherein said hair care composition is for hair care of a subject, wherein said subject is preferably a human or animal.

9. Use of the hair care composition of any of the preceding claims in the non-medical treatment of the hair of a subject, wherein said subject is preferably a human or animal.

10. The use of the following components A, B and C:
A) malic acid,
B) arginine, and
C) shikimic acid;
in the manufacture of a hair care composition,
wherein in said hair care composition each of said components A, B and C is comprised by <_ 0.5% by weight of said composition.

11. The use of claim 10, wherein in said hair care composition each of said components A, B and C is comprised by at least 0.01%, preferably at least 0.05%, further preferably at least 0.1% by weight of the composition.

12. The use of claim 10 or claim 11, wherein the mass ratio of said components A, B and C in said hair care composition in is selected from one of the following options a)-f):
option a)
| | | | | |
|---|---|---|---|---|
| A | : | B | : | C |
| 0.01-0.5 | | 0.01-0.5 | | 0.01-0.5 |
option b)
| | | | | |
|---|---|---|---|---|
| A | : | B | : | C |
| 0.05-0.5 | | 0.05-0.5 | | 0.05-0.5 |
option c)
| | | | | |
|---|---|---|---|---|
| A | : | B | : | C |
| 0.1-0.5 | | 0.1-0.5 | | 0.1-0.5 |
option d)
| | | | | |
|---|---|---|---|---|
| A | : | B | : | C |
| 0.01-0.2 | | 0.01-0.2 | | 0.01-0.2 |
option e)
| | | | | |
|---|---|---|---|---|
| A | : | B | : | C |
| 0.05-0.2 | | 0.05-0.2 | | 0.05-0.2 |
option f)
| | | | | |
|---|---|---|---|---|
| A | : | B | : | C |
| 0.1-0.2 | | 0.1-0.2 | | 0.1-0.2 |

13. The use of any of claims 10-12, wherein said components A, B and C are comprised in said composition in the identical % by weight, wherein preferably each of said components A, B and C is comprised in said composition by 0.5, 0.2 or 0.1 % by weight of said composition.

14. The use of any of claims 10-13, wherein said composition is formulated for application on a subject's hair.

15. The use of any of claims 10-14, wherein said composition is a formulation selected from the following: liquid, aerosol, suspension, solution, tincture, cream, paste, lotion, ointment, gel, balsam, foam, wherein said composition is preferably a shower gel and/or shampoo.
